# EUROPÄISCHE PATENTANMELDUNG

(11) **EP 1 127 562 A1**
(43) Veröffentlichungstag der Anmeldung: **29.08.2001**
(21) Anmeldenummer: 00124095.1
(22) Anmeldetag: 07.11.2000
(51) Int. Cl.: A61F 13/00, A61F 13/02, D04H 3/10

(54) **Medizinisches Verbandsmaterial**

(30) Priorität: 28.02.2000 DE 10009248
(71) Anmelder: Firma Carl Freudenberg, 69469 Weinheim (DE)
(72) Erfinder: Groitzsch, Dieter, Dr., 69493 Hirschberg (DE); Schaut, Gerhard, Dr., 69502 Hemsbach (DE)

(57) **Zusammenfassung**

Die Erfindung betrifft einen medizinisches Verbandsmaterial bestehend aus einem Mikrofilament-Vliesstoff mit einem Flächengewicht von 30 bis 150 g/m² und einer Reißfähigkeit von > 40 N/5cm besteht, bei dem der Vliesstoff aus schmelzgesponnenen, verstreckten und unmittelbar zu einem Vlies abgelegten Mehrkomponenten-Endlosfilamenten mit einem Titer von1,5 bis 5 dtex besteht und die Mehrkomponenten-Endlosfilamente gegebenenfalls nach einer Vorverfestigung zumindest zu 80 % zu Mikro-Endlosfilamenten mit einem Titer von 0,01 bis 1,0 dtex gesplittet und verfestigt sind.

## Beschreibung

Die Erfindung betrifft medizinisches Verbandsmaterial.

Als medizinische Verbandsmaterialen werden je nach Größe und Art der Wunde oder Verletzung Pflaster mit und ohne Wundkissen, Kompressen und Binden eingesetzt. Dabei dürfen die verwendeten Materialien zur Förderung des Heilungsprozesses keine Sperrwirkung gegen Wasserdampf und die beim Heilungsprozeß freigesetzten Gase, wie Kohlendioxyd, besitzen. Insbesondere bei Pflastern wird neben einer hohen Abriebbeständigkeit, eine Schmutz abweisende Oberfläche und eine hohe Taktilität, daß heißt, einem nubuklederartigen Griff, sowie eine Spalt- und Fusselfreiheit gefordert.

Aus den Dokumenten US 4,630,603; US 4,950,282; US 5,011,492 und US 5,679,190 sind medizinische Verbandsmaterialien bekannt, deren Trägermaterial aus Vliesstoffen bestehen. Nachteilig bei diesem Materialien ist, insbesondere wenn sie mit einem Haftkleber beschichtet sind, der eine sehr hohe Haftkraft auf menschlicher Haut besitzt, daß sie zu Vliesstoff-Schichtspaltungen beziehungsweise zum Herauslösen von Fasern neigen. Dadurch wird ihre Entfernbarkeit beim Verbandswechsel erschwert.

Die Erfindung hat sich die Aufgabe gestellt, ein medizinisches Verbandsmaterial anzugeben, welches eine hohe Abriebbeständigkeit, eine geringe Anschmutzbarkeit, eine hohe Textilität, Geschmeidigkeit und Anpassungsvermögen an Körperformen und Bewegungen, eine hohe Gas- und Wasserdampfdurchlässigkeit sowie keine Neigung zur Vliesschicht-Spaltung aufweist.

Erfindungsgemäß wird die Aufgabe durch ein medizinisches Verbandsmaterial gelöst, welches aus einem Mikrofilament-Vliesstoff mit einem Flächengewicht von 30 bis 150 g/m² und einer Reißfähigkeit von > 40 N/5cm besteht, wobei der Vliesstoff aus schmelzgesponnenen, verstreckten und unmittelbar zu einem Vlies abgelegten Mehrkomponenten-Endlosfilament mit einem Titer von 1,5 bis 5 dtex besteht und die Mehrkomponenten-Endlosfilamente gegebenenfalls nach einer Vorverfestigung zumindest zu 80 % zu Mikro-Endlosfilamenten mit einem Titer vom 0,01 bis 1,0 dtex gesplittet und verfestigt sind. Die medizinischen Verbandsmaterialien weisen keine Vliesstoff-Schichtspaltung auf und besitzen eine Wasserdampfdurchlässigkeit von mindestens 400 g/m² und 24 h.

Vorzugsweise ist das medizinische Verbandsmaterial eines, bei dem der Vliesstoff ein Flächengewicht von 40 bis 120 g/m² besitzt und aus schmelzgesponnenen, aerodynamisch verstreckten und unmittelbar zu einem Vlies abgelegten Mehrkomponenten-Endlosfilamenten mit einem Titer von 1,5 bis 3 dtex besteht und bei dem die Mehrkomponenten-Endlosfilamente gegebenenfalls nach Vorverfestigung zumindest zu 80 % zu Mikro-Endlosfilamenten von 0,05 bis 0,5 dtex gesplittet und verfestigt sind.

Besonders bevorzugt ist weiterhin ein medizinisches Verbandsmaterial, bei dem das Mehrkomponenten-Endlosfilament ein Bikomponenten-Endlosfilament ist, welches aus zwei inkompatiblen Polymeren besteht. Ein entsprechendes Bikomponenten-Endlosfilament setzt sich aus Polyestern, wie Polyäthylenterephthalat, Polybutylenterephthalat oder Polytetramethylenterephthalat und einem damit inkompatiblen Polymeren, wie Polypropylen, Polyethylen, Polyamid 6, Polyamid 6.6 oder einem thermoplastischen Elastomeren (TPE) zusammen. Für extrem weiche, nicht elastische Verbandsmaterialien wird als Polymerpaarung ein nicht-elastisches Polymer und ein TPE eingesetzt, wie zum Beispiel ein Polyester-Elastomer und ein Polyamid 6.6. Für extrem weiche, hoch dehnbare elastische medizinische Verbandsmaterialien wird eine Polymerpaarung aus zwei thermoplastischen Elastomeren eingesetzt, wie zum Beispiel aus einem Polyester-Elastomer und einem Styrol-Butadien-Styrol-Block-Copolymer. Weiterhin wird für starre, wenig dehnbare medizinische Verbandsmaterialien eine Polymerkombination aus nicht-elastischen Polymeren, sogenannten thermoplastischen Nicht-Elastomeren (TPNE) und thermoplastischen Elastomeren hergestellt. Bei der Polymerpaarung TPNE/TPE wird die niedriger schmelzende beziehungsweise erweichende Komponente zumindest zu teilflächigen Verbindungen der Filamente der höher schmelzende Komponente eingesetzt. Der Unterschied der Schmelztemperatur- beziehungsweise Klebetemperaturdifferenz zwischen TPNE und TPE beträgt mindestens 10°C, vorzugsweise mindestens 30°C. Für die Bindungen können die bekannten Bindeverfahren, wie gerichtete oder zirkulierende Heißluftströmungen, Heißkalandrierung oder Ultraschallverschweißung eingesetzt werden.

Besonders bevorzugt ist weiterhin ein medizinisches Verbandsmaterial, bei dem die Mehrkomponenten-Endlosfilamente einen Querschnitt mit orangenartiger Multisegmentstruktur aufweisen, wobei die Segmente alternierend jeweils eines der beiden inkompatiblen Polymeren enthalten und / oder eine "side-by-side"-Struktur besitzen. Das medizinisches Verbandsmaterial besitzt einen hohen Wasserdampfdurchgangsindex sowie eine Feuchtigkeitsaufnahmefähigkeit, so daß gegebenenfalls bei der Anwendung als Pflaster auch auf ein Wundkissen verzichtet werden kann. Die Unter- und Oberseite des medizinischen Wundverbandes muß nicht die gleiche Beständigkeit gegen mechanischen Abrieb im trockenen und feuchten Zustand aufweisen. Zur Verbesserung der Abriebbeständigkeit und Erzielung einer oberflächlich folienartigen Oberfläche der Oberseite, die weiterhin eine mikroporöse Struktur besitzt, wird das medizinische Verbandsmaterial durch Andrücken auf einer glatten, beheizten Walze verfestigt und abgeglättet.

Weiterhin ist das medizinische Verbandsmaterial eines, bei dem die beiden Seiten des Verbandmaterials eine unterschiedliche Segmentstruktur besitzen. Dadurch wird die Absorptionsfähigkeit des Verbandsmaterials erhöht. Weiterhin kann durch einen unterschiedlichen Grad der Verschlingung der Fasern untereinander die Absorptionsfähigkeit verbessert werden. Dabei wird die Wasserstrahlverfestigung auf der Ober- und Unterseite des medizinischen Verbandsmaterials mit unterschiedlichen Drücken vorgenommen, so daß eine Integritätserhöhung von der Ober- zur Unterseite eintritt. Die Verschlingungsintensität ist jedoch selbst an den schwächsten Querschnittsbereichen so intensiv, daß eine Spaltung Vliesstoffschichten im nassen und trockenen Zustand vollständig verhindert wird. Bei der Verwendung des Verbandsmaterials als Pflaster wird die Unterseite, auf die die Klebstoffbeschichtung aufgetragen, vorzugsweise nicht abgeglättet, da eine geringe Rauhigkeit eine bessere Verankerung des Klebstoffes bewirkt. Weiterhin können die Bikomponenten-Endlosfilamente des medizinischen Verbandsmaterials vor der Splittung mit an sich bekannten Methoden mit einer Kräuselung versehen werden, wobei die Kräuselung zweidimensional flach, zum Beispiel zick-zack-förmig oder spiralförmig, sein kann.

Besonders bevorzugt ist ein medizinisches Verbandsmaterial, bei dem mindestens eines der das Mehrkomponenten-Endlosfilament bildenden inkompatiblen Polymere Additive, wie Farbpigmente, permanent wirkende Antistatika, Fungizide, Bakterizide, Weichmacher, Stabilisatoren, Netz- und Trennmittel, optische Aufheller, die den Spinncharakter beziehungsweise Schmelzfluß beeinflußende Zusätze und / oder die hydrophilen oder hydrophoben Eigenschaften beeinflussende Zusätze in Mengen bis zu 10 Gew.-%, enthält. Je nach Art der Anwendung können so die spezifischen Anforderungen erfüllt werden. Die medizinisch wirksamen Zusätze werden dabei so gewählt, daß sie entweder bereits unmittelbar nach dem Ausspinnen oder nach einer Lagerung an die Faseroberfläche migrieren und sich dort anreichern, sowie an die Umgebung abgegeben werden können. Die Zusätze, die die physikalischen Eigenschaften der Fasern beeinflussen, werden vorzugsweise so gewählt, daß sie zeitlich gleichbleibend über den ganzen Mikrofaserquerschnitt statistisch verteilt sind.

Das Verfahren zur Herstellung eines medizinischen Verbandsmaterials besteht vorzugsweise darin, daß Mehrkomponenten-Endlosfilamente aus der Schmelze ersponnen, verstreckt und unmittelbar zu einem Vlies abgelegt werden, gegebenenfalls eine Vorverfestigung erfolgt und der Vliesstoff durch Hochdruck-Fluidstrahlen verfestigt sowie gleichzeitig in Mikro-Endlosfilamente mit einem Titer von 0,01 bis 1,0 dtex gesplittet wird. Die so hergestellten medizinischen Verbandsmaterialien weisen einen weichen Griff, eine hohe Abriebbeständigkeit und eine Wasserdampfdurchlässigkeit von mindestens 400 g/m² und 24 h, vorzugsweise von 800 g/m² und 24 h, auf.

Vorzugsweise besteht das Verfahren zur Herstellung des medizinischen Verbandsmaterials darin, daß die Verfestigung und Splittung der Mehrkomponenten-Endlosfilamente dadurch erfolgt, daß der gegebenenfalls vorverfestigte Vliesstoff mindestens einmal von jeder Seite mit Hochdruckfluidstrahlen beaufschlagt wird. Durch die Wahl der Anzahl der Hochdruckfluidstrahlenbehandlung und den dabei eingestellten Drücken läßt sich der gewünschte Gradient der Verschlingungsintensitäten und damit der Absorptionsfähigkeit einstellen.

Vorzugsweise ist das Verfahren zur Herstellung des medizinischen Verbandsmaterials weiterhin eines, bei dem die Färbung der Mehrkomponenten-Endlosfilamente durch Spinnfärben vorgenommen wird. Insbesondere bei Pflastern kann das medizinische Verbandsmaterial farblich in gewissen Grenzen der Hautfarbe des Patienten angepaßt und weiß, beige oder dunkelbraun eingefärbt werden. Alternativ dazu kann die Farbe auch auf die Oberseite des Verbandsmaterials aufgedruckt werden.

Vorzugsweise ist das Verfahren zur Herstellung des medizinischen Verbandsmaterials weiterhin eines, bei dem zwei Spinnbalken eingesetzt werden, von denen einer ein Mehrkomponenten-Endlosfilament mit einer "pie"-Segment-Struktur und das andere mit einer "side-by-side"-Segment-Struktur erzeugt. Insbesondere bei der Verwendung von Verbandsmaterialien ohne Wundkissen weist dieses Material eine gute Absorptionsfähigkeit auf.

Vorzugsweise ist das medizinische Verbandsmaterial eines, das zur Herstellung von Pflastern für flächige Abdeckung von kleinflächigen Schädigungen der Haut verwendet wird. Dazu wird das Material zumindest an der nicht mit Klebstoff applizierten Seite hydrophob, schmutzabweisend und gegebenenfalls auch alkoholabweisend ausgestattet. Vorzugsweise geschieht das dadurch, daß mindestens eines der beiden Mikrofaser-Endlosfilamente die genannten Eigenschaften aufweist. Zur Erhöhung der Schmutz- und Alkoholabweisung kann mindestens einem der Ausgangspolymere eine Fluorcarbonharz zugesetzt werden. Alternativ ist auch die nachträgliche Applikation durch Vollbadimprägnierung, Streichen, Bedrucken, Pflatschen oder Besprühen, vorzugsweise mit wässrigen Dispersionen möglich. Besonders bevorzugt ist aus wirtschaftlichen Gründen die sogenannte Naß-in-Naß-Vollbadtränkung. Dadurch kann auf eine Zwischentrocknung zur Entfernung der nach der Wasserstrahlbehandlung und -absaugung auf der Ware verbleidender Feuchte verzichtet werden. Unter Naß-in-Naß-lmprägnierung wird ein Verfahren verstanden, bei dem die feuchte Ware ohne Zwischentrocknen mit dem Ausrüstungsmittel versehen wird. Dabei werden weiterhin Maßnahmen ergriffen, die im Fall der Vollbadimprägnierung in einem Foulard eine Verdünnung der Flotte im Foulard-Trog verhindern. Vorteilhafterweise geschieht das dadurch, daß die Konzentration beziehungsweise der Feststoffgehalt des zum Foulard-Trog ständig zugepumpten Ausrüstungsmittels höher ist, als die Flotte im Trog. Weiterhin können dem Ausrüstungsmittel zusätzlich ein textiler Farbstoff und / oder ein Pigment-Farbstoff zugesetzt werden. Im Falle eines Pigment-Farbstoffes wird für dessen Anbindung eine wäßrige Kunststoffdispersion zugegeben. Als vorteilhaft für die gleichzeitige Hydrophobierung und Färbung von Polyesterfasern im erfindungsgemäßen medizinischen Verbandsmaterial hat sich eine Kombination nichtionischer Fluorcarbonharz-Dispersion / Emulsion und im Thermosol-Verfahren auftragbarer Dispersionsfarbstoffe erwiesen.

Zur Herstellung eines Pflasters wird das aus Mikro-Endlosfilamenten bestehende Trägervlies mit einem Haftkleber beschichtet, der schon bei Hauttemperatur eine Fixierung durch leichten Andruck auf der Haut des Patienten ermöglicht. Als Klebstoffauftragsmethoden sind Filmextrusion, Bedruckung, Bepuderung, Beflockung, Bespinnung oder Besprühung geeignet.

Vorzugsweise erfolgt eine vollflächige Klebstoffbeschichtung mit einem Haftkleber, der eine hohe Wasserdampf- sowie Sauerstoff- und Kohlendioxyd-Durchlässigkeit besitzt.

Vorzugsweise beträgt die Wasserdampfdurchlässigkeit des mit Klebstoff beschichteten Vliesstoffes mindestens 400 g/m² und 24 h, vorzugsweise jedoch mindestens 800 g/m² und 24 h.

Vorzugsweise wird der Kleber, in einem organischen Lösungsmittel gelöst oder in Wasser dispergiert, aufgetragen. Alternativ dazu kann der Kleber auch über eine sogenannte Umkehrbeschichtung (reverse coating) zuerst auf ein Release-Medium (Release-Papier) aufgetragen werden und dann mit diesem zusammen auf das Mikrofilament-Vlies transferiert werden. Weiterhin ist auch eine Release-Papier freie Applikation von Haftkleber auf dem Mikrofilament-Vlies dann vorteilhaft, wenn dieses auf der Klebstoff abgewandten Seite eine Release-Ausrüstung trägt.

Vorzugsweise besteht eine solche Klebstoff-Release-Ausrüstung aus in an sich bekannten Hydrophobierungsmitteln, wie beispielsweise fettsauer modifizierten Melaminharzen und deren Verschnitten mit Paraffinen, Schwermetallsalz-vernetzten Paraffinen, Fluorcarbonharzen, Silikonen oder Silikonelastomeren beziehungsweise Kombinationen der vorgenannten Produkte. Das medizinische Verbandsmaterial kann in Form von Schmalrollen mit und ohne Vliespapier eingesetzt werden. Zur Abdeckung von sekretierenden Wunden wird auf die mit Haftkleber beschichtete Seite noch teilflächig ein absorbierender Körper zur Sekret- und Blutaufnahme aufgebracht. Der absorbierende Körper, im weiteren Wundkissen genannt, ist auf der Wundseite beziehungsweise Hautseite mit einem porösen, abgeglätteten Vliesstoff, einer perforierten Folie oder ein Plastiknetz zur Verhinderung einer Verklebung mit der Wunde beziehungsweise Verhinderung einer Traumatisierung der Wunde beim Entfernen des Verbandsmaterials abgedeckt. Diese Abdeckung kann das absorbierende Wundkissen entweder umschlingen, so daß auch die Schnittkante des Saugkissens umhüllt und auf der Klebeschicht fixiert ist. Weiterhin kann sie auch nur auf dem Wundkissen aufliegen und mit diesen durch mechanische oder adhesive Bindetechniken verbunden sein.

Vorteilhafterweise ist das Wundkissen mit Keim tötenden oder Keimwachstum hemmenden Ingredienzien imprägniert. Das Wundkissen kann aus einem saugfähigen Gewebe, Gewirke, einem Vliesstoff, einer Vliesstoff-Faden-Kombination oder einem offenzelligen Schaumstoff bestehen.

Vorzugsweise besteht das Wundkissen aus hydrophilen oder hydrophilisierten Fasern, Schaumstoffen oder Gelen, wie Alginat, carboxymethylierten Lyocell-Fasern oder Chitosan.

Vorzugsweise ist das medizinische Verbandsmaterial weiterhin eines, das zur Herstellung wirkstoffhaltigen Pflastern verwendet wird. Durch Einkapselung von medizinischen Wirkstoffen beziehungsweise durch Imprägnierung des Wundkissen mit medizinischen Wirkstoffen können wirkstoffhaltige Pflaster erzeugt werden, die eine Depotwirkung des Wirkstoffes, das heißt eine Langzeitapplikation des Wirkstoffes besitzen.

Vorzugsweise ist das medizinische Verbandsmaterial weiterhin eines, das zur Herstellung von Bandagebinden verwendet wird. Vorteilhafterweise wird das Verbandsmaterial einem mechanischen Weichmachungsprozeß unterzogen. Besonders günstig ist eine Reckung des Vliesstoffes in Längs- und / oder Querrichtung, vorzugsweise in Querrichtung, das heißt im 90°-Winkel zu den Wasserstrahlgassen. Dadurch kann die Elastizität des Materials erhöht werden.

Vorzugsweise wird das medizinische Verbandsmaterial zur Herstellung von Wundkompressen verwendet. Durch Zusatz von Bindemitteln können entsprechend steife Wundkompressen erhalten werden. Weiterhin können bei Pflastern oder Wundkompressen auch zusätzlich eine weitere Schicht aus Stapelfasern aufgebracht werden. Dadurch kann die Absorptionsfähigkeit des Verbandsmaterials verbessert.

Die Wahl der Rohstoffkomponenten für das erfindungsgemäße medizinische Verbandsmaterial richtet sich nach der Sterilisationsanforderungen. Vorzugsweise wird die Sterilisation durch Bestrahlung vorgenommen, wobei beim Einsatz von Polypropylen dieses mit entsprechenden Stabilisatoren gegen den Abbau versehen wird.

Die Erfindung wird an den nachfolgenden Beispielen näher erläutert:

### Beispiel 1

Es wurde mit bekannter Technik Bikomponenten-Endlosfilamente aus den Komponenten Polyäthylenterephthalat und Polyamid 6.6 ersponnen, die nach dem Verlassen der Düsenplatte und vor dem Ablegen auf einem Ablageband mit gerichteten Luftströmung abgeschreckt und zu feinen Filamenten verstreckt worden sind. Das Ablegen der Endlosfilamente zu einem Flächengebilde erfolgte in der Weise, daß in Maschinenlaufrichtung und in Querrichtung zur Maschinelaufrichtung annähernd die gleichen mechanischen Festigkeiten resultieren (isotrope Faserverteilung). Die Verteilung der beiden faserbildenden Polymere erfolgte, im Querschitt der Faser betrachtet, abwechselnd in Form von Küchenstücken aus Polyäthylenterephthalat und Polyamid 6.6, wobei von insgesamt 16 Küchenstücken jeweils 8 aus Polyäthylenterephthalat und 8 aus Polyamid 6.6 bestehen. Die Massengewichtsverhältnisse von Polyäthylenterephthalat / Polyamid 6.6 in der Bikomponentenfaser mit der 16er-Küchenstückaufteilung im Querschnitt beträgt 65 / 35. Die Bikomponentenfaser weist einen Titer von ca. 2,0 dtex auf. Dem Polyamid 6.6-Anteil wurde ein Antistatikum zugesetzt. Der Splittfaseranteil aus Polyäthylenterephthalat enthielt keine weitere Zusätze, wie z.B. Farbstoff und/oder Hydrophilierungsmittel.

Der bis dahin ungebundene Spinnvliesstoff mit einem Flächengewicht von 76 g/m² wurde durch Pressung zwischen kalten Walzen zum Zwecke eines erleichterten Weitertransportes verdichtet und einer Hochdruckwasserstrahl-Vernadelungseinrichtung zugeführt. Mit steigenden Wasserstrahldrücken von 70 bis 400 bar in Maschinenlaufrichtung wurde die Endlosfilamente des Spinnvliesstoffes zunächst intensiv miteinander verschlungen und schließlich gegen Ende der Hochdruckwasserstrahlpassage zu ultrafeinen Mikrofasern mit Kuchenstück-Formstruktur im Querschnitt mit einem durchschnittlichen Titer von 0,125 dtex aufgesplittet. Der Aufsplittungsgrad betrug - bestimmt durch optische Auszählung unter dem Rasterelektronenmikroskop - ca. 96 %. Anschließend wurde bei einer Trocknertemperatur von 180°C getrocknet und bei gleicher Temperatur ein Flächenschrumpf von 14,2 % ausgelöst, der fast ausschließlich in einem Breitenverlust der Ware führt. Das Flächengewicht erhöht sich dabei von 76 auf 88,6 g/m².

Die getrocknete Ware wurde im Vollbadverfahren mit einer Mischung aus nichtionisch eingestelltem Fluorcarbonharz und anionisch eingestellten Dispersionsfarbstoffen getränkt. Anschließend wurde getrocknet, das Fluorcarbonharz vernetzt und gleichzeitig der Dispersionsfarbstoff bei 190°C in einem Thermosol-Verfahren fixiert. Die Aufnahme an Farbstoff und Fluorcarbonharz bezogen auf die Festsubstanz betrug 0,50 g/m². Die relativ niedrigen Farbechtheiten der Dispersionsfarbstoffe auf dem Polyamid 6.6-Mikrofaseranteil des Endlosfilamentvliesstoffes stören nicht, da es sich bei der Anwendung als Träger für Pflaster oder Wundschnellverbände um einen Einmalartikel (Disposable) mit diesbezüglich nicht so hohen Farbechtheitsforderungen handelt.

Es resultierte ein stark hydrophobes und alkoholabweisendes Trägermaterial für Pflaster bzw. Wundschnellverbände mit sehr hoher mechanischer Beständigkeit gegen Abrieb, sehr hoher Festigkeit, absolut keiner Gefahr einer Delaminierung (keine Vliesstoffspaltung), einem Mikrofasergriff sowie guter Drapierfähigkeit und Textilität.

In der folgenden Tabelle sind die arithmethischen Mittelwerte der gemessen Prüfdaten zusammengestellt:

| Merkmal | Prüfnorm | Einheit | Arithmethischer Mittelwert |
|---|---|---|---|
| Flächengewicht | EN 29073-01 | g/m² | 89 |
| Dicke | DIN 53855-01 | mm | 0,49 |
| Luftdurchlässigkeit bei 200 Pa | EN ISO 9237 | dm³/sec m² 150 | |
| Höchstzugkraft längs | EN 29073-03 | N/50 mm | 258,6 |
| Höchstzugkraft quer | EN 29073-03 | N/50 mm | 222,3 |
| Dehnung bei Höchstzugkraft längs | EN 29073-03 | % | 49 |
| Dehnung bei Höchstzugkraft quer | EN 29073-03 | % | 52 |
| Abriebbeständigkeit naß bei 2N und 40 Touren | Freudenberginterne Methode | Note | Seite 1: 1,5 |
| | | | Seite 2: 1,0 |
| Hydrophobizität nach Cobb | DIN EN 20535 | g/m² | Seite 1: 6 |
| | | | Seite 2: 6 |

## Patentansprüche

1. Medizinisches Verbandsmaterial bestehend aus einem Mikrofilament-Vliesstoff mit Flächengewichten von 30 bis 150 g/m² und einer Reißfähigkeit von > 40 N/5cm besteht, wobei der Vliesstoff aus schmelzgesponnenen, verstreckten und unmittelbar zu einem Vlies abgelegten Mehrkomponenten-Endlosfilamenten mit einem Titer von 1,5 bis 5 dtex besteht und die Mehrkomponenten-Endlosfilamente gegebenenfalls nach einer Vorverfestigung zumindest zu 80 % zu Mikro-Endlosfilamenten mit einem Titer von 0,01 bis 1,0 dtex gesplittet und verfestigt sind.

2. Medizinisches Verbandsmaterial nach Anspruch 1, dadurch gekennzeichnet, daß der Vliesstoff mit Flächengewichten von 40 bis 120 g/m² aus schmelzgesponnenen, aerodynamisch verstreckten und unmittelbar zu einem Vlies abgelegten Mehrkomponenten-Endlosfilamenten mit einem Titer von 2 bis 3 dtex besteht und die Mehrkomponenten-Endlosfilamente gegebenenfalls nach einer Vorverfestigung zumindest zu 80 % zu Mikro-Endlosfilamenten mit einem Titer von 0,05 bis 0,5 dtex gesplittet und verfestigt sind.

3. Medizinisches Verbandsmaterial nach Anspruch 1 oder 2, dadurch gekennzeichnet, daß das Mehrkomponenten-Endlosfilament ein Bikomponenten-Endlosfilament ist, welches aus zwei inkompatiblen Polymeren besteht.

4. Medizinisches Verbandsmaterial nach einem der Ansprüche 1 bis 3, dadurch gekennzeichnet, daß die Mehrkomponenten-Endlosfilamente einen Querschnitt mit orangenartiger Multisegment-Struktur aufweisen, wobei die Segmente alternierend jeweils eines der beiden inkompatiblen Polymeren enthalten und/oder eine "side-by-side"-Struktur besitzen.

5. Medizinisches Verbandsmaterial nach Anspruch 4, dadurch gekennzeichnet, daß die beiden Seiten des medizinischen Verbandsmaterials eine unterschiedliche Segment-Struktur besitzen.

6. Medizinisches Verbandsmaterial nach einem der Ansprüche 1 bis 5, dadurch gekennzeichnet, daß mindestens eines der das Mehrkomponenten-Endlosfilament bildenden inkompatiblen Polymere Additive, wie Farbpigmente, permanent wirkende Antistatika, Fungizide, Bakterizide, Weichmacher, Stabilisatoren und/oder die hydrophilen oder hydrophoben Eigenschaften beeinflussende Zusätze in Mengen bis zu 10 Gewichtsprozent, enthält.

7. Verfahren zur Herstellung eines medizinischen Verbandsmaterials nach einem der Ansprüche 1 bis 6, dadurch gekennzeichnet, daß Mehrkomponenten-Endlosfilamente aus der Schmelze ersponnen, verstreckt und unmittelbar zu einem Vlies abgelegt werden, gegebenenfalls eine Vorverfestigung erfolgt und der Vliesstoff durch Hochdruck-Fluidstrahlen verfestigt sowie gleichzeitig in Mikro-Endlosfilamente mit einem Titer von 0,01 bis 1,0 dtex gesplittet wird.

8. Verfahren nach Anspruch 7, dadurch gekennzeichnet, daß die Verfestigung und Splittung der Mehrkomponenten-Endlosfilamente dadurch erfolgt, daß der gegebenenfalls vorverfestigte Vliesstoff mindestens einmal von jeder Seiten mit Hochdruck-Fluidstrahlen beaufschlagt wird.

9. Verfahren nach Anspruch 7 oder 8, dadurch gekennzeichnet, daß die Färbung der Mehrkomponenten-Endlosfilamente durch Spinnfärben vorgenommen wird.

10. Verfahren nach einem der Ansprüche 7 bis 9, dadurch gekennzeichnet, daß zwei Spinnbalken eingesetzt werden, von denen einer Mehrkomponenten-Endlosfilamente mit einer "Pie"-Segmentstruktur und der andere mit einer "side-by-side"-Segmentstruktur erzeugt.

11. Medizinisches Verbandsmaterials nach einem der Ansprüche 1 bis 10, dadurch gekennzeichnet, daß er zur Herstellung von Pflastern für die Abdeckung von kleinflächigen Schädigungen der Haut verwendet wird.

12. Medizinisches Verbandsmaterials nach einem der Ansprüche 1 bis 10, dadurch gekennzeichnet, daß er zur Herstellung von wirkstoffhaltigen Pflastern verwendet wird.

13. Medizinisches Verbandsmaterials nach einem der Ansprüche 1 bis 10, dadurch gekennzeichnet, daß er zur Herstellung von Pflastern für den Verschluß von Platz-, Schnitt- oder Operationswunden verwendet wird.

14. Medizinisches Verbandsmaterials nach einem der Ansprüche 1 bis 10, dadurch gekennzeichnet, daß er zur Herstellung von Bandage-Binden verwendet wird.

15. Medizinisches Verbandsmaterials nach einem der Ansprüche 1 bis 10, dadurch gekennzeichnet, daß er zur Herstellung von Wundkompressen verwendet wird.
